# EUROPEAN PATENT APPLICATION

(11) **EP 4 014 843 A1**
(43) Date of publication of application: **22.06.2022**
(21) Application number: 20315491.9
(22) Date of filing: 16.12.2020
(51) Int. Cl.: A61B 5/00, A61B 5/0205, A61B 5/0215, A61B 5/024, A61B 5/026, A61B 5/145, A61B 5/1455, A61B 5/1459, A61B 17/00

(54) **MEDICAL IMPLANT AND METHOD OF MEASURING A PARAMETER**

(71) Applicant: HoliStick Medical, 75003 Paris (FR)
(72) Inventor: Pouletty, Christophe, 75005 Paris (FR); Pau, Antoine, 75005 Paris (FR); Corot, Claire, 69006 Lyon (FR); Roche, Ellen, Galway (IE); Berrebi, Alain, 92130 Issy-Les-Moulineaux (FR); Settergren, Magnus, 11 333 Stockholm (SE); Keillor, Matthew, 75010 Paris (FR); Warnak, Boris, 4104 Oberwil (CH); Bruneau, Maelle, 75011 Paris (FR); Quintin, Mathieu, 78120 Rambouillet (FR); Sanchez, Sandrine, 75012 Paris (FR)
(74) Representative: Hepp Wenger Ryffel AG

(57) **Abstract**

The invention relates to a medical implant (1), preferably a patch (7). The medical implant (1) is adapted to repair or close a defect (D). In particular, the defect may be an opening in a ventricular or atrial or vessel wall (W). The medical implant (1) comprises at least one sensor (14, 15, 16, 18', 18") and an actor (17, 19, 21, 22, 23).

## Description

The present invention relates to a medical implant and a method of measuring a parameter according to the preamble of the independent claims.

Defects in tissue, for example atrial septal defects (ASD) or ventricular septal defects (VSD), are a fairly common condition in humans that is typically treated minimally invasively. Such defects can cause a variety of symptoms such as shortness of breath and a higher burden on the heart and lungs.

As a consequence, a myriad of implantable devices have been proposed in the prior art, many of which can be deployed in a minimally invasive way.

For example, closure of atrial septal defects (ASD) by deploying umbrella-like implants through a catheter have been disclosed by Lock et al. (DOI: 10.1161/01.CIR.79.5.1091).

However, known implants exhibit several disadvantages. Typically, they are attached to a tissue wall mechanically. On the one hand, this can lead to small, for example local and/or focal, injuries of the tissue to be treated. On the other hand, mechanical attachment places constraints on the material choice and mechanical strength of the components employed. Finally, mechanical anchoring can also be difficult to realize in a minimally invasive manner.

In addition, it can be difficult to determine parameters of the surrounding environment of the implant. Oftentimes, monitoring parameters such as proper placement of the implant, implant geometry due to stress and strain on the implant, but also physiological parameters such as blood values, blood pressure, 02 saturation, heart values, temperature, inflammation etc. require expensive and difficult techniques.

Thus, the object of the present invention is to overcome the drawbacks of the prior art, in particular to provide a medical implant, and a method of determining a parameter that enables easier, cheaper, and safer implantation and monitoring of an implant and its surrounding.

This and other objects are achieved by the medical implant and the method according to the characterizing portion of the independent claims of the invention.

The medical implant according to the invention is preferably a patch. A patch can, in particular, be an implant with a substantially flat shape which is designed to be attached to a surface such as a tissue wall. Preferably, it is mechanically flexible such that it can adapt to an underlying surface shape or structure. Particularly preferably, the patch is made of a polymeric material or of pericardium. The medical implant is adapted to repair or close a defect. In particular, the defect may be an opening in a ventricular or atrial or vessel wall. According to the invention, the medical implant comprises at least one of a sensor and an actor.

In a preferred embodiment, the medical implant according to the invention may be adapted to close a cavity such as a left atrial appendage. Preferably, the cavity may be closed by attaching the implant at an ostium of the cavity. Additionally or alternatively, the medical implant may be adapted to close a defect in a tissue wall, for example a patent foramen ovale (PFO).

While detailed functionalities will be disclosed in the context of specific features below, the general functionality of the sensor and the actor are explained here.

The sensor is adapted to measure at least one parameter of or related to the implant and/or of the region surrounding the implant. For example, the sensor may be adapted to measure stress/strain/shear/force/oscillation frequency, and/or motion/displacement/bending of the implant, attachment of cells and/or other tissue to the implant and/or quantitatively a thickness of a tissue layer formed on the implant, but also hemodynamic parameters (flow properties, turbulence, temperature, pressure, inflammation values, O₂ saturation, levels of cholesterol, platelets, sugar, lipids) or tissue parameters (electric voltage, temperature, inflammation value, stiffness, conductivity, composition etc.).

The sensor may also be adapted to detect platelet activation and/or thrombosis and/or blood coagulation. Particularly preferably, the sensor may be adapted such that it detects thrombosis and/or coagulation on two sides of the implant. In this case, a medical person may accept (i.e. not treat) thrombosis/coagulation on one side of the implant, but take action if thrombosis/coagulation forms on the other side of the implant. For example, a thrombus on one side of the patch may further occlude a defect and thus be desired, while a thrombus on the other side of the implant may have negative consequences for the treatment.

Pressure measurements may be used to determine, for example, atrial cardiomyopathy, ASD in patients, or heart failure.

The sensor may also detect arrhythmia and/or atrial fibrillation, in particular by a pressure and/or voltage-based measurement. It may also monitor biocolonization/endocolonization/cell migration /endocardialization/epithelialization/endothelialisation/fibrous capsule formation/tissue ingrowth.

In particular in cases where the presence of blood is to be detected, the sensor may also be adapted to detect the presence of liquids. Preferably, the presence of liquids is detected by a reflectivity measurement, by measurement of an electrical resistance, and/or by having a soluble marker (for example a salt) whose presence can be detected.

Particularly preferably, the sensor is adapted to detect a parameter that represents the proper placement and attachment of the implant to tissue, in particular during and after delivery, and as such to monitor and diagnose if said placement/attachment is or becomes insufficient. Additionally or alternatively, the sensor may detect the geometry of the implant, in particular during and after delivery.

It will be understood that the sensor may be adapted to measure any other parameter during and/or after implantation as well. In particular, the collected data may be transferred/read during an implantation ("live") or after implantation.

The actor may be adapted to perform an action based on, or in assistance to, measurements of the sensor. For example, the actor may act on the tissue by providing a force, heat, cooling, or an electrical voltage in order to enable a particular measurement of the sensor. Additionally or alternatively, the actor may provide an action in response to measurement of the sensor. For example, the actor may release a drug or other bioactive substance (or trigger the release) depending on a measurement value. Additionally or alternatively, the actor may conduct RF ablation based on a detection of arrhythmia by the sensor. It may also send the measurement parameters to a receiver outside of the body. In this context, it may also receive a signal from outside of the body and process it, for example to trigger a measurement by the sensor.

The actor may, additionally or alternatively, be adapted to release a drug. In particular if the sensor is adapted to detect coagulation/thrombosis, the actor may be adapted to release an anti-thrombogenic agent to prevent strokes. Alternatively, in particular if the sensor also measures a blood sugar value, the actor may be adapted to release insulin.

However, the actor may be adapted to release any other drug as well based on a sensor measurement. The actor may also release the drug independently of the sensor's measurement.

By incorporating an actor and a sensor, the medical implant according to the invention provides a particularly simple way of determining, for example, if the medical implant is properly attached at the desired location and if the body is reacting in a desired or undesired way (such as inflammation) to the implant. The actor further enables corrective action, for example by changing the shape of the medical implant. The treatment also becomes safer because the ease of monitoring enables simple,fast and continuous determination if a medical intervention is necessary.

The actor may also be a battery or a capacitor that provides electrical energy to the sensor.

Additionally or alternatively, the sensor may be adapted to measure a residual shunt, for example a residual shunt through an occluder device.

The implant is preferably adapted in size and shape to close an opening in a ventricular and atrial wall, for example a patent foramen ovale (PFO). Typically, a medical implant for such an application has a substantially round, in particular circular, shape, though any shape such as a triangle, square, or more complicated shapes is possible. It is substantially flat, and has a typical diameter of 20-25 mm. It may of course be larger or smaller, depending on a patient or the opening to be treated. For example, an opening in the heart of a child may be smaller, requiring a diameter as small as 10 mm. It may also be as large as 30 mm, for example if a patient is very tall, or if the defect is particularly large for other reasons. Typically, the thickness of the medical implant is 100-200 µm. Of course, the thickness may also be adapted and be as thin as 50 µm or as thick as 500 µm.

The medical implant can comprise an adhesive composition. Preferably, the medical implant further comprises two states. In the first state, the medical implant can be deployed to the implant site while the adhesive composition is inactive. The medical implant can be brought in a second state, in particular at the implant site, by an activation mechanism. The adhesive composition is curable by a curing mechanism in said second state.

An adhesive is a particularly preferred variant to attach the medical implant to a tissue wall as it is safe, easy to implement and requires comparatively little in-vivo manipulation. However, the person skilled in the art will understand that other attachment mechanisms would work equally well. In particular, the sensor and actor may provide similar benefits regardless of how exactly the implant is attached to the tissue.

Preferably, in the first state, the medical implant comprises at least one cavity that contains the adhesive composition. It may be adapted to release the adhesive composition in the second state.

A cavity shall be understood as a preferably closed structure in the medical implant that can retain another substance such as a liquid, a viscous liquid, or even a solid. For example, the cavity may be a large hollow structure or a small pore. Cavities are particularly advantageous to store the adhesive composition in the first state because the adhesive is then protected from the surrounding media, in particular humidity from the body. Thus, it does not accidentally engage tissue before activation, and prevents problem while deploying the medical implant, such as clogging of a catheter due to adhesive leaks.

Preferably, in the first state, the medical implant comprises a plurality of cavities, in particular milli-sized or micro-sized cavities.

Micro-sized cavities shall be understood as cavities with any shape that have characteristic size in the micrometer range, i.e. from 1 µm to 1000 µm. For example, the medical implant may comprise a plurality of spherical cavities with a diameter of 10 µm to 100 µm. This is particularly advantageous because it ensures a homogenous distribution, and if necessary mixing, of the adhesive composition when it is released.

Preferably, the at least one cavity comprises an additive that, upon exposure to humidity, swells. The at least one cavity may be adapted to release the adhesive composition upon swelling.

This enables a particularly easy way of releasing the adhesive, because exposure to blood automatically makes the cavities swell and thus releases the adhesive.

Preferably, the medical implant comprises at least two different kinds of cavities.

The two types of cavities may be different in size, composition, shape, or any other property. For example, the two different types of cavities may contain different additives that make them swell at different rates. They may also have different wall thickness, different radii, or be made of a different material. They may also be adapted such that one type of cavity swells and the other does not. This enables better control of the release of the adhesive. For example, one component can selectively be released first, or one component can be released at a different rate than the other. It may also enable a better control of the rate of release if the adhesive only comprises one component. For example, it may be advantageous to release a first fraction of the adhesive, and release a second fraction at a later point. It is also possible to adapt the cavities such that they release the adhesive composition or a component thereof at different pressures or temperatures.

Preferably, the at least two different types of cavities are adapted to contain different components of an adhesive composition, in particular in a liquid, gel, dried, or gaseous form. This may, in particular, include any of the features of two different types of cavities as described above. However, it may also be possible to conceive particular properties that enable the storage of a particular component of an adhesive. For example, a certain wall material may be particularly advantageous for one component of an adhesive composition, but may be incompatible with another. Thus, it may be advantageous to adapt the cavities to the specific components of an adhesive composition. Of course, it may also be possible to provide different sizes or degradation rates of the cavities to account for desired ratios of two components in the final (mixed) adhesive composition.

Preferably, the adhesive composition comprises two components that are individually disposed in the cavities, such that in the first state, the two components are separated. This enables the controlled mixing in the second state, for example at the implant site. This is of course particularly advantageous for twocomponent adhesives that are not curable before mixing. In this case, unplanned curing before adhesive release, for example by accidental exposure to humidity or elevated temperature can be prevented. However, it is of course also conceivable to have an adhesive that comprises one component that only additionally hardens the adhesive composition, for example through crosslinking. It may be advantageous to dispose such a component separately as well.

Preferably, the adhesive composition is adapted to be curable upon mixing of the at least two components. This prevents accidental curing before release of the adhesive. Any curing mechanism to cure the curable adhesive composition after mixing is conceivable. It may be an increase in temperature, exposure to humidity, exposure to electromagnetic radiation such as visible light, infrared light, ultraviolet light, or a combination thereof.

Preferably, the adhesive composition is adapted to spontaneously cure upon mixing of the at least two components. This offers a particularly advantageous way of deploying the adhesive composition because it does not require additional processing steps beyond the release and the spontaneous mixing of the two components. However, it is of course possible to combine such an adhesive composition with additional curing mechanisms. For example, an adhesive composition with two components may spontaneously cure upon mixing, but exposure to an additional curing mechanism such as electromagnetic radiation, humidity, or increased temperature may accelerate the curing if necessary. It is also conceivable that a third component is used as an additional hardener.

Preferably, the cavities are adapted to release the adhesive composition upon a temperature increase, in particular an increase to the temperature of a human body. This enables the automatic release of the adhesive composition after implantation because the implant is heated up to 37°C.

Preferably, the cavities are adapted to release the adhesive composition upon exposure to electromagnetic radiation. For example, the cavities may degrade or burst upon irradiation with visible light, infrared light, ultraviolet light, or similar. They may also be adapted to release the adhesive composition upon irradiation with a particular wavelength or wavelength range. If more than one type of cavity is present, they may also be adapted to release the adhesive upon irradiation with different wavelength ranges, such that it is possible to selectively release one component at a time. In general, cavities that are adapted to release the adhesive composition upon exposure to electromagnetic radiation are particularly advantageous because they enable the combination with a delivery device that can transmit light for the release, for example delivery devices as disclosed in WO 2015/175662 A1. It is also a particularly safe way of releasing the adhesive composition because light is typically not prevalent in the human body, especially at the short wavelengths (UV/VIS), thus preventing accidental release.

Preferably, the cavities are adapted to release the adhesive composition upon an increase in pressure. In particular, this enables the release of the adhesive composition upon inflation of a balloon. However, any other mechanism to provide a pressure increase is also conceivable. For example, the cavities may be adapted such that exposure to a liquid causes swelling due to an osmotic pressure that provides a pressure difference. It may also comprise a separate inflation reservoir to provide a pressure on the cavities, the implant, or another part thereof.

Preferably, the cavities are adapted to release the adhesive composition upon a mechanical compression. For example, a compression by a balloon of a delivery device may be employed and is particularly advantageous because delivery devices with balloons are known and thus easy to implement.

Preferably, the adhesive composition is adapted to be curable by exposure to electromagnetic radiation. This provides the similar advantages already described in the context of cavities that are adapted to release upon exposure to electromagnetic radiation. For example, the adhesive composition may be curable upon irradiation with visible light, infrared light, ultraviolet light, or similar. It may also be adapted to be curable upon irradiation with a particular wavelength or wavelength range. In general, adhesive compositions that are adapted to release the adhesive composition upon exposure to electromagnetic radiation are particularly advantageous because they enable the combination with a delivery device that can transmit light for the release, for example delivery devices as disclosed in WO 2015/175662 A1. It is also a particularly safe way of curing the adhesive composition because light is typically not prevalent in the human body, thus preventing accidental curing.

Preferably, the sensor comprises at least one electrode. The sensor may also comprise an array of electrodes. In particular, the electrode may be adapted to measure a heartbeat. Additionally or alternatively, the electrode may also measure electrical currents and/or voltages and/or electrical resistance/impedance/conductance between two points on the medical implant and/or tissue. For example, a change in resistance may indicate a change in tissue properties, the overgrowth of tissue over medical implant, or the presence of blood (or lack thereof) .

The sensor can also comprise a photodetector. A photodetector may, in particular, be used to detect reflected or transmitted light, for example emitted by a catheter. Alternatively, the photodetector may also detect light emitted by the actor. Data on transmission, absorption and/or reflection of light may be used to detect the presence of biomarkers, drugs, hormones, or other biological molecules. A transmission/absorption/reflection measurement may also be used to measure the 02 saturation in the blood and/or blood flow in a microvasculature, for example to quantify a level of vascularization of neotissue. A reflection measurement is also particularly suited to measure the attachment surface area of the implant and to detect a possible detachment, because the reflectivity of the interface between tissue and implant differs from the reflectivity of the interface between the implant and blood/vacuum/gas.

The sensor can also comprise a piezoelement. The piezoelement can be adapted to detect stress or strain on the implant. Additionally or alternatively, the piezoelement may also be used to measure a pressure, for example a blood pressure and/or a pressure acting on the implant.

The sensor may also comprise at least one temperature sensing element. The temperature sensing element may detect only changes in temperature, or measure an absolute value of temperature. Alternatively, it may only detect if the temperature is above or below a certain threshold value.

The sensor may comprise at least one ultrasound transducer, in particular a Doppler transducer. An ultrasound transducer is particularly suited to measure flow of blood and changes in tissue structure.

Preferably, the sensor is adapted to measure at least one of a pressure of a fluid (in particular blood pressure and/or atrial pressure), conductance (of blood, tissue and/or the implant), impedance, resistance, optical density, optical absorbance, velocity of flow (in particular of blood, for example to detect stasis), acceleration (for example to detect septal, atrial, or ventricular movement), oxygen saturation of blood (i.e. oxymetry), or a blood value (such as brain natriuretic peptide, hormones, presence of certain cells, atrial fibrillation). Detection of brain natriuretic peptide (BNP) may be used as a prealarm for heart failure. BNP levels in blood can be increased due to heart failure. In addition, BNP levels can correlate a level of ventricular wall stress and severity of heart failure. Similarly, the the sensor may be adapted to detect the presence and/or a level of matrix metallopeptidase (MMP) and/or tissue inhibitor of metalloproteinase (TIMP).

In addition, it is conceivable that the medical implant may comprise a pressure valve, for example a pressure valve that opens into the right atrium at a threshold pressure. Thus, a pressure valve may be introduced between the two atria to relieve a left atrial pressure, in turn relieving the pulmonary pressure.

Measurement of an impedance value may be linked to a degree of coverage by cell layers, and thus linked to level of endothelialization of the implant.

An impedance and/or conductance value may also vary depending on the type of tissue growing on an implant and therefore allow a determination of the type of tissue. Such a determination has been described by Giurgiutiu et al. (J. Invest. Surg., 17(5):257-270; DOI: 10.1080/08941930490502835) which is incorporated herein by reference.

Tissue conductance and/or impedance may also indicate a level of vascularization.

The medical implant can have a first and a second surface. The first surface faces a tissue and is attached or attachable to said tissue when implanted as intended. The second surface is arranged on an opposite side of the first surface. The implant comprises at least one pressure-sensitive element arranged on the first surface. The pressure-sensitive element may, in particular, be adapted to detect an adhesive fore between the tissue and the medical implant.

Such pressure-sensitive elements enable a particularly easy determination of whether or not the implant is securely attached to the tissue.

Preferably, the pressure-sensitive element is part of the actor. The pressure-sensitive element may, however, alternatively be part of the sensor.

Particularly preferably, the pressure-sensitive element comprises a spring or a spring-like mechanism that provides a force between the tissue and the implant. Measuring or otherwise determining the force provides a minimum adhesion force. In addition, the detection of adhesion can be performed, for example, by measurement of the size/degree of compression of the pressure-sensitive element by the sensor. Additionally or alternatively, the sensor may detect an area of the contact surface between the implant and the tissue in the vicinity of the pressure-sensitive element.

A pressure-sensitive element in particular enables to determine an adhesive force between the implant and tissue qualitatively or quantitatively.

The sensor may also be adapted to measure a contact surface between the medical implant and biological tissue. For example, it may measure the total contact surface, or the contact surface relative to the total surface area of the implant, or relative to the maximum theoretical surface area.

Preferably, the measurement of the contact surface is performed by light reflection and/or measurement of an electrical resistance, or impedance between markers on an implant surface and a reference electrode stuck in the tissue, or between markers placed between the implant and the tissue.

Additionally or alternatively, the sensor may be adapted to measure a contact force between the medical implant and biological tissue.

The medical implant may also comprise at least one marker. Preferably, the marker is a radio-opaque and/or echo-opaque marker.

Markers enable a particularly easy imaging of the position and shape of the implant, for example by X-ray imaging of echography. If multiple markers are present, the distance between these markers in relation to an ideal value (such as the distance in the absence of mechanical stress) can be used to determine stress or strain in the medical implant. The radio-opaque markers may also be aligned along a certain geometry. Spacing/movement of the radio-opaque markers may thus provide information of adhesion and/or patch placement and, in particular, enable easier and/or more precise positioning.

Echo-opaque markers may be used in addition to or as an alternative of radio-opaque markers. Echo-opaque markers enable visibility in echographical techniques.

Optical coherence tomography and photoacoustic methods are preferred methods of imaging and/or controlling the implant.

Therefore, preferably, the medical implant comprises at least two markers. The sensor may be adapted to detect a distance and/or orientation between the at least two markers relative to one another.

Thus, the stress and strain in the implant and/or its position and orientation may be measured by the sensor.

Preferably, the medical implant comprises a communication device. In particular, the communication device may be an RFID chip. The communication device can be brought in operable connection with an external device to transmit measurement data gathered by the at least one sensor. Additionally or alternatively, the communication device may comprise an active component, i.e. a device that is able to emit information. Preferably, an active component is based on micro-acoustics.

In particular, the external device may be a computer and comprise a scanner, an antenna, or another RFID reader.

In a preferred embodiment, the actor and/or the communication device may be adapted to transfer data, in particular an adhesive force, to a delivery device which comprises a gauge and/or display to display the data, in particular adhesive force.

The invention further relates to a method of measuring a parameter. The medical parameter includes at least one of a pressure, conductance, impedance, resistance, optical density, optical absorbance, velocity of flow, acceleration, oxygen saturation of blood, degree of vascularization, size and/or presence and/or type of a tissue/scar/capsule, and a blood value inside a patient's body, in particular a patient's heart. The parameter is measured by a sensor comprised by an implant implanted in the patient's body, in particular the patient's heart. The parameter is transmitted wirelessly to a receiver outside the patient's body. Additionally or alternatively, the data may be used in an internal feedback loop, for example to correct a parameter (such as mechanical deformation) of the implant.

The person skilled in the art will understand that the medical implant according to the invention is particularly adapted to perform the method according to the invention. Conversely, the method according to the invention is preferably performed using a medical implant according to the invention.

In the following, the invention is described in detail with reference to the following figures, showing:
- Fig. 1a-1c:: schematically different embodiments of adhesive delivery suitable for the invention.
- Fig. 2a-2b:: an alternative embodiment of an adhesive delivery for a medical implant.
- Fig. 3a-3b:: a patch with capsules containing a filler material.
- Figs. 4a-4d:: alternative embodiments of an implant according to the invention.
- Figs. 5a-5b:: two alternative embodiments of an implant according to the invention.
- Figs. 6a-6c:: the mechanism of the sensor and actor of the implant of Fig. 5a.
- Fig. 7:: schematically an implant according to the invention when implanted.

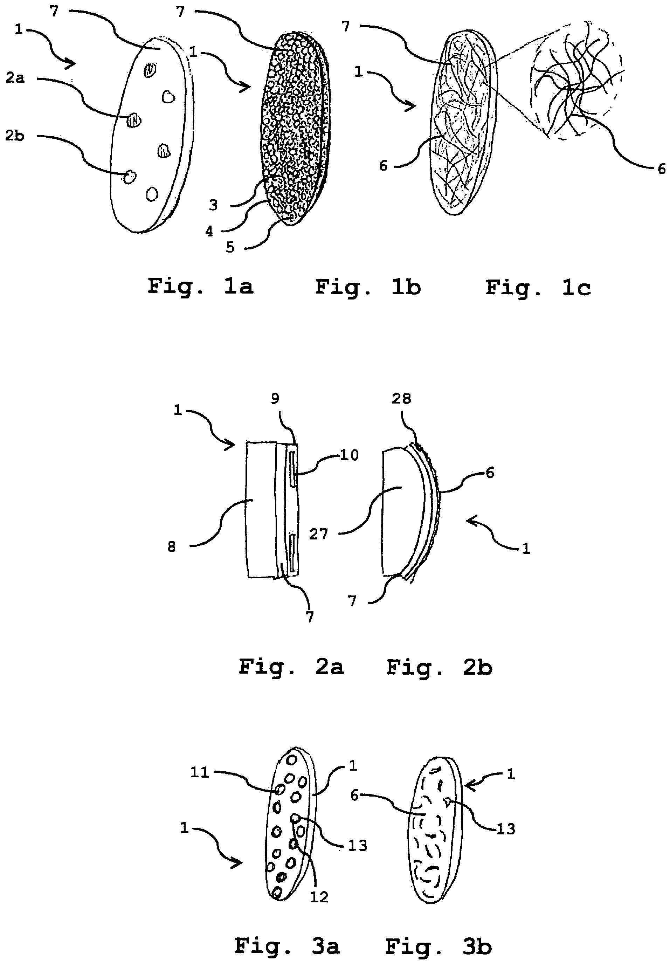

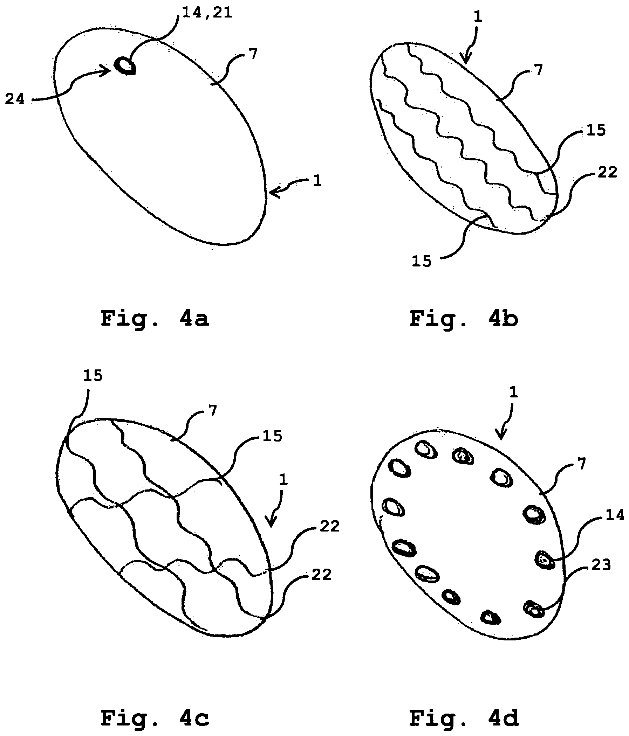

Figs. 1a-1c show schematically different embodiments of implants 1 comprising an adhesive composition. For simplicity, the embodiments of Figs. 1-3 are shown without a sensor and/or actor and only illustrate possible adhesive delivery mechanisms that are suitable with an implant according to the invention. The implants 1 of Figs. 1a-1c are configured as patches. The person skilled in the art will understand that the embodiments shown in Figs. 1-3 can be combined with any actor and sensor as shown herein and in particular in the Figs. 4-7.

Fig. 1a shows an implant 1 comprising two different types of cavities 2a, 2b. The cavities 2a, 2b are spherical and have a diameter of approximately 1 mm. The implant 1 has a diameter of 20 mm and consists of poly(lactic acid-co-glycolic acid). Other non-absorbable materials such as PLA- GA, PLGA, PCL, PU could also be used. The cavities 2a, 2b contain two different components, a resin and a hardener, of an adhesive composition. The resin and the hardener are physically separated from one another and become curable upon mixing. Thus, the adhesive composition is not curable in the shown state where the two components are separated. However, cavities 2a, 2b in the shown embodiment are adapted to burst upon application of a mechanical pressure, for example exerted by an inflatable balloon. The burst of the cavities causes the release of both components of the adhesive composition, rendering it curable. Typical adhesives might be GelMA (Metacrylated Gelatin), CollMA (methacrylated Collagen), tropoelastin, or MeTro (methacrylated tropoelastin).

Fig. 1b shows an implant 1 that comprises a foam 3. Pores 5 of the foam are surrounded by walls 4 made of a hydrogel. The hydrogel is adapted to degrade in the human body within 24 months. The pores 5 are filled with an adhesive composition. The implant 1 is adapted to release the adhesive composition from the pores 5 upon a mechanical deformation. The adhesive composition here is curable by exposure to electromagnetic radiation. It will be understood by the person skilled in the art that any adhesive composition could be combined with the shown implant 1, in particular any curing mechanism. The pores 5 are adapted in their size to facilitate cell in-growth such that after release of the adhesive composition, the empty pores can serve as a scaffold for tissue growth. The biodegradation of the implant 1 is adapted such that the implant 1 degrades after the formation of new tissue.

Fig. 1c shows yet another embodiment of an implant 1. The shown implant is made of electrospun fibers 6 of polycaprolactone. The fibers have a diameter of approximately 3 µm and a length of several 100 µm. The fibers are coated with methacrylated gelatin as an adhesive composition. This implant can thus easily be attached to tissue and is particularly easy to fabricate by electrospinning. It will be understood by the person skilled in the art that the fibers could also consist of an adhesive composition instead of being coated by it. Similarly, although polycaprolactone is particularly advantageous for electrospinning, the fibers could be made of another material.

Figs. 2a-2b show an embodiment of medical implant 1 in a lateral cross-section. The implant 1 comprises an inflation reservoir 8, a patch 7, and a separate layer 9 comprising reservoirs 10 for containing an adhesive.

Fig. 2a shows the medical implant 1 in a first state. The inflation reservoir is empty. The reservoirs 10 for comprising an adhesive are filled with adhesive. Typically the adhesive can be a poly(acrylic) acid which uses an acrylate/methacrylate/amuse crosslinker.

The patch 7 in this embodiment is made of electrospun fibers of Dacron and is not biodegradable. However, it would of course be possible to adapt the implant 1 to be biodegradable as well. The layer 9 comprising the reservoirs 10 for the adhesive is of solid Dacron.

Fig. 2b shows the medical implant 1 in a second state wherein the inflation reservoir 8 is inflated. The inflation of the inflation reservoir 8 applies a mechanical pressure to the reservoirs 10 containing the adhesive which is subsequently squeezed out, forming a layer of adhesive 6 on one side of the implant 1. Here, the patch 7 is adapted to not be penetrable by the adhesive composition, thus leading to a selective release of the adhesive composition on the other side of the implant. The inflation reservoir is adapted to be removed after inflation. However, it would also be conceivable to form it from an implant grade material that remains in the patient.

Fig. 3a and 3b show an embodiment of an implant 1 comprising capsules 36 with a filler material 37 in the capsule wall 38.

Fig. 3a shows the implant 1 in a first state. The capsules 11 are spherically shaped and have a diameter of approximately 0.5-2 mm and are evenly distributed in the implant 1. An adhesive composition 12 comprising methacryloyl-substituted tropoelastin is contained in the inside of the capsules 11. Here, the capsules 11 are adapted to break open due to an osmotic pressure. For example, exposure to blood or another liquid causes swelling of the capsules 11. The resulting pressure increase then causes bursting of the capsules 11.

Fig. 3b shows the implant 1 in a second state after rupture of the capsules 11. The adhesive composition 12 is evenly distributed over the surface of the implant 1. A filler material 13 (e.g. a hydrogel and/or a protein-based material such as collagen) remains in the adhesive composition 12 and provides additional mechanical strength to the adhesive layer.

Fig. 4a shows an embodiment of a medical implant 1 according to the invention. The implant 1 is configured as a patch 7. It comprises a unit 24 which incorporates both a sensor 14 and an actor 21. The sensor comprises a piezoelement (not visible) and a voltage meter which measures the blood pressure. Additionally or alternatively, the sensor may be adapted to measure an impedance. Optionally, the sensor may also comprise an electroconductive polymer to enable or support such measurements. The actor comprises a battery and a processor (not visible) to process sensing signal of the piezoelement. The device 24 further comprises an RFID chip that stores the processed data of the actor 21. It is thus possible to access the data without a surgical intervention.

Fig. 4b shows an alternative embodiment of an implant 1 according to the invention. The implant is configured as a patch 7 and comprises two wires 15 acting as sensors and arranged on one surface of the patch 7. The wires exhibit a piezoeffect and thus create a voltage if they are deformed. The sensor wires 15 are electrically connected to a third wire 22 that acts as an actor. If the patch is deformed, the voltage created by the sensors 15 causes a deformation of the actor 22 and thus self-corrects the shape of the patch 7. In an alternative embodiment, only sensor wires may be used, i.e. a deformation can be detected without a self-correcting force being induced by an actor.

Fig. 4c shows a similar embodiment to the implant 1 of Fig. 4b. Here, the implant comprises two sensor wires 15 and two actor wires 22. They are arranged as pairs of one sensor 15 and one actor each that are operatively connected as described in the context of Fig. 4b. The pairs are arranged at an angle with respect to each other. Thus, the shape self-corrects against deformation in two separate directions. It would also be possible to use any number of sensor 15 and/or actor 22 wires together to achieve a different correction. Additionally or alternatively, several pairs (or groups) of sensor/actor wires 15, 22 can be used at any desired angle.

Fig. 4d shows yet an alternative embodiment of a patch 7. The shown embodiment is similar to the one shown in Fig. 4a. However, the present embodiment comprises a plurality of sensors 14 and actors 23 arranged in proximity of the circumference of the patch 7. For simplicity, only one sensor 14 and actor 23 each are designated with a reference sign. Six sensors 14 and six actors 23 are arranged in an alternating pattern. The sensors are adapted to measure the distance and the angle between the two neighbouring actors 23 and its own position. The data acquired by the sensor is saved and transmitted e.g. by an RFID chip (not shown for simplicity).

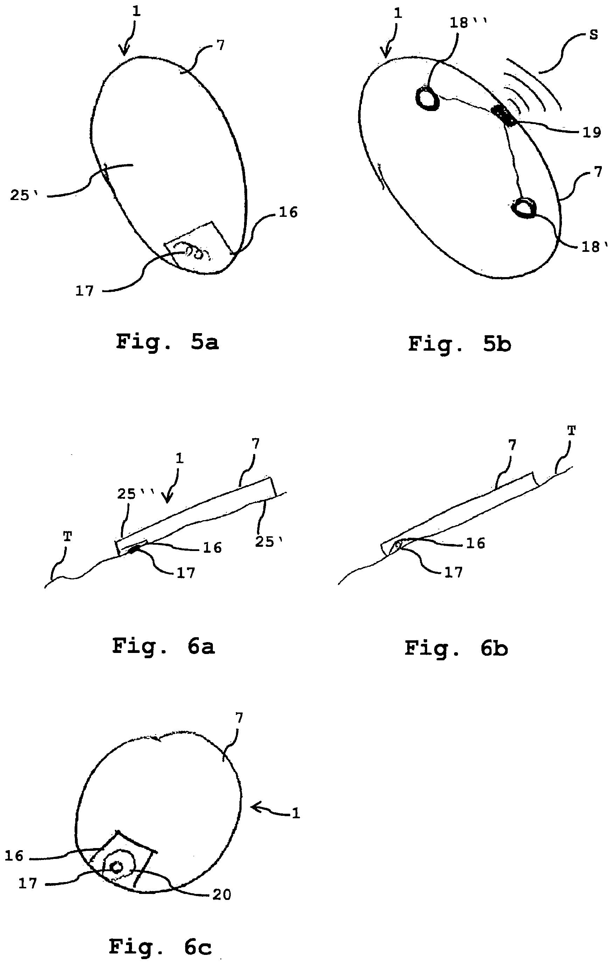

Fig. 5a shows an alternative embodiment of a medical implant 1 according to the invention. A sensor 16 is configured as a photodetector with an integrated battery. An actor 17 is formed by a spring. Both the sensor 16 and the actor 17 are arranged on a first surface 25' of the patch 7 that is intended to be attached to a heart wall (not shown). When attached, the spring of the actor 17 is compressed. The spring 17 is designed such that a correctly placed and attached patch 7 cannot be displaced by the spring force, i.e. the intended adhesion force is stronger. If, however, the patch 7 is not properly attached to the tissue wall, the spring will displace the patch 7, and in particular the surface of the sensor 16, so that it does not touch the tissue wall. The sensor 16 comprises a light-emitting device (LED, not shown) that illuminates the surface of the patch 7. The photodetector of the sensor 16 generates a signal that represents the reflectivity of the interface between the tissue (not shown) and the surface of the patch 7. If the patch 7 is detached from the tissue, the interface and thus its reflectivity change. Therefore, the measurement of the reflectivity is a parameter that represents the proper attachment of the implant 1 to the tissue.

Fig. 5b shows an alternative embodiment of an implant 1. The implant comprises two electrodes 18',18" that can detect the resistance or impedance of an underlying tissue when attached to a tissue wall (not shown). The electrodes may, additionally or alternatively, also be adapted to measure other electrical parameters, for example currents, voltages, potentials, and fields. In particular, the electrodes may also detect a heartbeat. The actor 19 comprises a battery and a processing unit and is adapted to send a signal S if the values measured by the sensor 18',18" are higher or lower than a saved threshold value. For example, the actor may transmit the signal S to a patient's smartphone or smartwatch or a system of a caregiver and alert the patient or caregiver of abnormal values.

Figs. 6a-6c schematically illustrate the working principle of the implant of Fig. 5a.

Fig. 6a shows the implant 1 attached a tissue wall T. The implant 1 is configured as a patch 7 and shown in a properly attached position. Therefore, the spring 17 is compressed and the photodetector 16 is pressed against the tissue. The first surface 25' with the spring 17 and the sensor 16 is arranged against the tissue T. A second surface 25" is arranged on an opposite side of the first surface 25'.

Fig. 6b shows the implant 1 of Fig. 6a after the implant has started to detach from the tissue T. The spring displaces the surface of the implant 1 up from the tissue T. As mentioned above, the spring is designed to not exert a force sufficient to detach a properly attached patch 7. However, if the patch 7 is not properly attached to the tissue T, the actor 17 causes the patch 7 to first detach in the proximity of the actor 17, as shown presently.

Fig. 6c shows the same scenario as Fig. 6b but from a top perspective. In the vicinity of the actor 17, the patch 7 is detached from the tissue. Thus, the reflectivity in its surrounding changes, which change can be measured by the sensor 16. Thus, a possible detachment can be detected before the implant 1 fails.

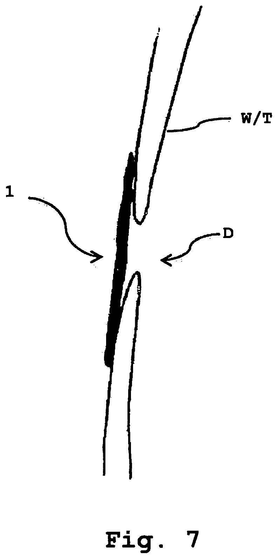

Fig. 7 schematically shows an implant 1 according to the invention attached to a tissue T. The tissue shown here is a septal wall W with a defect D, presently a patent foramen ovale. The medical implant 1 is designed in its size and shape to close said defect D.

It will be understood that the embodiments disclosed herein are of exemplary nature. All features described in the context of the embodiments above can be freely combined.

## Claims

1. A medical implant (1), preferably a patch (7), that is adapted to repair or close a defect (D), preferably an opening in a ventricular or atrial or vessel wall (W), wherein
the medical implant (1) comprises at least one of a sensor (14, 15, 16, 18', 18") and an actor (17, 19, 21, 22, 23).

2. A medical implant (1) according to claim 1, wherein the medical implant (1) comprises an adhesive composition (12), and
the medical implant (1) comprises two states, wherein the medical implant (1) in its first state can be deployed to the implant site while the adhesive composition (12) is inactive, and brought into the second state, preferably at the implant site, by an activation mechanism,
the adhesive composition (12), in said second state, being curable by a curing mechanism.

3. A medical implant (1) according to one of the preceding claims, wherein the sensor comprises at least one electrode (18',18"), preferably an array of electrodes.

4. A medical implant (1) according to one of the preceding claims, wherein the sensor comprises at least one photodetector (16).

5. A medical implant according to one of the preceding claims, wherein the sensor comprises at least one piezoelement.

6. A medical implant according to one of the preceding claims, wherein the sensor (14, 15, 16, 18', 18") comprises at least one temperature sensing element.

7. A medical implant according to one of the preceding claims, wherein the sensor comprises at least one ultrasound transducer, in particular a Doppler transducer.

8. A medical implant according to one of the preceding claims, wherein the at least one sensor (14, 15, 16, 18', 18") is adapted to measure at least one of a pressure of a fluid, conductance, impedance, resistance, optical density, optical absorbance, velocity of flow, acceleration, oxygen saturation of blood, or a blood value.

9. A medical implant (1) according to one of the preceding claims, wherein the implant has a first (25') and a second surface (25"), and wherein the first surface (25') faces a tissue (T) and is attached or attachable to said tissue (T), and the second surface (25") is arranged on an opposite side of the first surface (25'), wherein the implant (1) comprises at least one pressure-sensitive element (17) arranged on the first surface (25'), in particular a pressure-sensitive element (17) adapted to detect an adhesive force between the tissue (T) and the medical implant (1).

10. A medical implant (1) according to one of the preceding claims, wherein the sensor (16) is adapted to measure a contact surface between the medical implant (1) and biological tissue (T).

11. A medical implant (1) according to one of the preceding claims, wherein the medical implant (1) comprises at least one marker, preferably a radio-opaque and/or an echo-opaque marker.

12. A medical implant (1) according to claim 11, comprising at least two markers, wherein the sensor is adapted to detect a distance and/or an orientation of the at least two markers relative to one another.

13. A medical implant (1) according to one of the preceding claims, further comprising a communication device (19), in particular an RFID chip, which can be brought in operable connection with an external device to transmit measurement data gathered by the at least one sensor (14, 15, 16, 18', 18").

14. A method of measuring a parameter, wherein the medical parameter includes at least one of a pressure, conductance, impedance, resistance, optical density, optical absorbance, velocity of flow, acceleration, oxygen saturation of blood, degree of vascularization, size and/or presence and/or type of a tissue/scar/capsule, and a blood value inside a patient's body, preferably inside a patient's heart, wherein the parameter is measured by a sensor (14, 15, 16, 18', 18") comprised by an implant (1) implanted in the patient's body, preferably the patient's heart, **characterized in that** the parameter is transmitted wirelessly to a receiver outside the patient's body.
